Europäisches Patentamt

European Patent Office

Office européen des brevets

(19)

(11) Veröffentlichungsnummer : **0 070 366**
**B1**

(12) **EUROPÄISCHE PATENTSCHRIFT**

(45) Veröffentlichungstag der Patentschrift :
28.08.85

(51) Int. Cl.⁴ : **C 12 Q   1/28, G 01 N 33/53**

(21) Anmeldenummer : **82104215.7**

(22) Anmeldetag : **14.05.82**

(54) **Verfahren zum Nachweisen von okkultem Humanblut in Humanstuhlproben.**

(30) Priorität : **16.07.81 CH 4673/81**

(43) Veröffentlichungstag der Anmeldung :
**26.01.83 Patentblatt 83/04**

(45) Bekanntmachung des Hinweises auf die Patenterteilung : **28.08.85 Patentblatt 85/35**

(84) Benannte Vertragsstaaten :
**AT BE CH DE FR GB IT LI NL SE**

(56) Entgegenhaltungen :
EP-A- 0 017 661
EP-A- 0 032 782
DE-A- 2 820 310
US-A- 4 016 043
THE JOURNAL OF IMMUNOLOGY, Band 116, Nr. 6,
Juni 1976, Seiten 1554-1559, Williams & Wilkins CO.,
USA, K. KATO et al.: "Enzyme-linked immunoassay:
conjugation of the Fab' fragment of rabbit IgG with
beta-D-galactosidase from E. coli and its use for
immunoassay"
CLINICAL CHEMISTRY, Band 22, Nr. 6, 1976, Seiten
733-738, S.L. SCHARPE et al.: "Quantitative enzyme
immunoassay: Current status"

(73) Patentinhaber : **F. HOFFMANN-LA ROCHE & CO.
Aktiengesellschaft**
**CH-4002 Basel (CH)**

(72) Erfinder : **Gallati, Harald, Dr.**
**Ingelsteinweg 13**
**CH-4143 Dornach (CH)**

(74) Vertreter : **Kloter, Rudolf et al**
**Postfach 3255 Grenzacherstrasse 124**
**CH-4002 Basel (CH)**

## Beschreibung

Die vorliegende Erfindung betrifft ein Verfahren zum Nachweis von okkultem Humanblut in Humanstuhlproben.

Der Nachweis von okkultem Blut im Stuhl stellt eine anerkannte Methode zur Früherfassung von Carcinomen des Kolons dar. Eine alte, bewährte und einfache Suchmethode ist der chemische Nachweis von okkultem Blut im Stuhl. Hierbei beruht das Testprinzip auf der Peroxidase-Wirkung von Hämoglobin, wobei in Gegenwart von Wasserstoffperoxid der Indikator (Benzidin, o-Toluidin, Guajak) oxidiert wird und eine Blaufärbung resultiert. Es handelt sich hierbei allerdings um eine unspezifische Reaktion, da neben Hämoglobin auch andere Stuhlkomponenten Peroxidase-Aktivität besitzen, wie Nahrungsmittel, vor allem Blutwürste, rotes Fleisch sowie Pflanzenbestandteile und Darmbakterien. Bei den kommerzialisierten Tests zum Nachweisen von okkultem Blut im Stuhl, welche in Form von Testbriefchen erhältlich sind, wird denn auch der Patient gebeten, drei Tage vor Testbeginn und während der Testperiode auf den Verzehr von rohem und halbrohem Fleisch sowie von Wurstwaren (z. B. Tartar, Steak, Leber, Salami, Blutwurst) zu verzichten.

Diese Vorsichtsmassnahmen sind für den Patienten umständlich und vermögen falsch positive Resultate nicht ganz auszuschliessen, was zeitaufwendige und kostspielige Nachuntersuchungen, wie Koloskopie oder Rektoskopie, erforderlich macht.

Aus diesem Grunde wurde auch schon vorgeschlagen (Songster, C.L. et al. in American Cancer Society 1099ff, 1980), bei einem kerkömmlichen, mit Guajak imprägnierten Testbriefchen, eine zusätzliche Probenaufnahmestelle anzubringen, die nicht mit Guajak imprägniert ist, und deren Inhalt im Falle einer positiven Guajakprobe mit Hilfe einer immunologischen Methode auf die Anwesenheit von Humanhämoglobin zu untersuchen.

Diese Methode hat jedoch den Nachteil, dass diese weitere Stuhlprobe mit den Stuhlproben, die dem Guajaktest unterworfen werden, nicht unbedingt identisch ist, indem sie z. B. einer anderen Stuhlentleerung bzw. einer anderen Stelle des Stuhles entnommen wurde, was wiederum zu Unsicherheiten führen kann.

Es besteht demnach ein Bedürfnis, Stuhlproben, die dem Peroxidasetest unterworfen wurden, auf die Anwesenheit von Humanhämoglobin, Humanalbumin oder Humanglobulin zu untersuchen, die normalerweise in Humanstuhl nicht vorkommen.

Da die peroxidatische Methode mit Hilfe einer äthanolischen Peroxidlösung durchgeführt wird, war man bisher der Ansicht, dass diese Methode die immunologischen Eigenschaften solcher Komponenten zerstört.

Demgegenüber wurde nun im Rahmen der vorliegenden Erfindung überraschenderweise gefunden, dass dies nicht zutrifft, sondern dass es ohne weiteres möglich ist, in einer Stuhlprobe, die vorgängig der Peroxidase-Methode unterzogen wurde, einen immunologischen Nachweis von Humanhämoglobin, Humanalbumin oder Humanglobulin durchzuführen.

Die vorliegende Erfindung betrifft demnach ein Verfahren zum Nachweis von okkultem Humanblut in Humanstuhlproben nach der Peroxidase-Methode, welche dadurch gekennzeichnet ist, dass anschliessend an die Durchführung dieser Methode in derselben Stuhlprobe Humanhämoglobin, Humanalbumin oder ein Humanglobulin immunologisch nachgewiesen wird.

Wie bereits erwähnt, kommen bei der Peroxidase-Methode diejenigen Verfahren in Betracht, bei denen z. B. Benzidin, o-Toluidin oder Guajak als Indikator benutzt wird.

Als Humanglobulin kommt Immunglobulin G, Immunglobulin M oder Immunglobulin A in Betracht.

Der immunologische Nachweis dieser Komponente kann nach jeder in der Immunologie bekannten Methode erfolgen. Als besonders bevorzugte Methode hat sich das Sandwich-Verfahren erwiesen. Bei diesem Verfahren wird die nachzuweisende Komponente mit Hilfe von zwei spezifischen Antikörpern bestimmt, von denen einer an eine Festphase gebunden ist und der andere mit einer Markierung versehen ist. Die verwendeten Antikörper können aus unterschiedlichen Tierseren gewonnen werden, doch können auch verschiedene monoklonale Antikörper verwendet werden. Als Festphase haben sich Kunststoffkügelchen als besonders geeignet erwiesen. Als Markierung kommen alle in der Immunologie bekannten Markierungsmittel in Betracht, wobei jedoch Enzyme besonders geeignet sind. Vorzugsweise wird als Enzym Peroxidase verwendet.

Der immunologische Nachweis dieser Komponente kann aber auch mit einem Latex-Agglutinationstest erfolgen.

Die nachfolgenden Beispiele erläutern die Erfindung.

### Beispiel 1

Humanstuhl wird mit 0 ml, 1 ml, 2 ml, 4 ml, 8 ml und 16 ml Humanblut pro 100 g frischer Stuhl versetzt und homogenisiert. Von diesen Stuhlproben wird je eine Spatelspitze in die beiden Vertiefungen eines Testbriefchens « Colo-Rectal-Roche-Test® »* aufgetragen, die Briefchen werden verschlossen und während zwei Tagen bei Raumtemperatur aufbewahrt.

Zum Nachweis von okkultem Blut im Stuhl wird nun die Lasche « Nur vom Arzt zu öffnen » entfernt und dann zuerst ein Tropfen einer Lösung von 0,1 M Citratpuffer mit 20 % Aethanol (pH 5) und anschliessend ein Tropfen einer 3 %igen $H_2O_2$-Lösung in Aethanol auf die Stuhlprobe gegeben und nach 30 Sekunden die entstandene Farbintensität (0 bis 4 +) abgeschätzt.

Für den nachfolgenden enzym-immunologischen Nachweis von Humanglobulin im Stuhl wird ein 9 mm breiter Streifen mit den beiden Stuhlflecken vom Testbriefchen abgeschnitten und dieser in 5 ml 0,1 M $NaHCO_3$ vom pH 9,5 während 30 Minuten bei 37 °C inkubiert. Nach gutem Mischen wird dann von dem klaren, geruchlosen, braun-gelb gefärbten Extrakt 0,1 ml in ein Kunststoff-Teströhrchen ($\emptyset$ = 1 cm) pipettiert, 0,1 ml 0,2 M Natriumphosphatpuffer vom pH 6,5 zugemischt, eine mit (Schaf) Anti-human-Globulin sensibilisierte Polystyrolkugel ($\emptyset$ = 6,35 mm) zugesetzt und 1 Stunde bei 37 °C inkubiert.

Nach Abtrennung des nicht gebundenen Materials wird die Kugel nochmals während 1 Stunde bei 37 °C mit 0,2 ml (Ziegen) Anti-human-Globulin-Peroxidase, gelöst in 0,2 M Natriumphosphatpuffer vom pH 6,5 mit 20 % Ziegenserum und 2 g/l Rinderserum-Albumin, inkubiert. Nach dreimaligem Waschen mit destilliertem Wasser wird die Kugel zum Nachweis der immunologisch gebundenen Peroxidase während 30 Minuten bei Raumtemperatur in 0,5 ml Substratpuffer (0,1 M Citratpuffer vom pH 5,0 mit 6 mM $H_2O_2$ und 40 mM o-Phenylendiamin) inkubiert. Nach Abstoppen der enzymatischen Reaktion durch Zugabe von 2 ml einer 1 N HCl wird zur Ermittlung des Gehaltes an Humanglobulin die Absorptionsdifferenz bei der Wellenlänge 492 nm ($\Delta A_{492nm/RT/30Min.}$) photometrisch bestimmt. Die Resultate sind in der Tabelle I zusammengefasst.

Tabelle I

| Testbriefchen: | Okkultblut-Test (Farbintensität) | Enzym-immunologischer Test ($\Delta A_{492\ nm/RT/30\ Min.}$) |
|---|---|---|
| Ohne Stuhlprobe (Reagenzienleerwert) | O | 0,000 |
| Stuhlprobe ohne Humanblut | O | 0,080 |
| Stuhlprobe mit 1 ml Humanblut pro 100 g Stuhl | O | 0,300 |
| Stuhlprobe mit 2 ml Humanblut pro 100 g Stuhl | O | 0,410 |
| Stuhlprobe mit 4 ml Humanblut pro 100 g Stuhl | 2 + | 0,680 |
| Stuhlprobe mit 8 ml Humanblut pro 100 g Stuhl | 4 + | 0,950 |
| Stuhlprobe mit 16 ml Humanblut pro 100 g Stuhl | 4 + | 1,530 |

Aus der Tabelle I geht die Sensitivität des enzymimmunologischen Okkultblut-Tests hervor, da die Absorptionsdifferenz abhängig von der Konzentration von Humanblut signifikant ansteigt.

### Beispiel 2

Humanstuhl wird mit je 4 ml a) Humanblut, b) Ziegenblut, c) Rinderblut, d) Kaninchenblut, e) Pferdeblut sowie mit 10 mg Meerrettich-Peroxidase pro 100 g frischer Stuhl versetzt und homogenisiert. Von diesen Stuhlproben wird je eine Spatelspitze in die beiden Vertiefungen des Testbriefchens « Colo-Rectal-Roche-Test® » aufgetragen, die Briefchen werden verschlossen und während einem Tag bei Raumtemperatur aufbewahrt.

Zum Nachweis von okkultem Blut im Stuhl wird nun die Lasche « Nur vom Arzt zu öffnen » entfernt und zuerst ein Tropfen einer Lösung von 0,1 M Citratpuffer mit 20 % Aethanol (pH 5) und anschliessend ein Tropfen einer 3 %igen $H_2O_2$-Lösung in Aethanol auf die Stuhlprobe gegeben und nach 30 Sekunden die entstandene Farbintensität (0 bis 4 +) abgeschätzt.

Für den nachfolgenden enzym-immunologischen Nachweis von Humanglobulin in den Stuhlproben wird ein 9 mm breiter Streifen mit den beiden Stuhlflecken vom Testbriefchen abgeschnitten und dieser in 5 ml 0,1 M $NaHCO_3$ vom pH 9,5 während 30 Minuten bei 37 °C inkubiert. Nach gutem Mischen wird vom klaren, rot-braungefärbten Extrakt 0,1 ml in ein Teströhrchen pipettiert, 0,1 ml 0,2 M Natriumphosphatpuffer vom pH 6,5 zugemischt, eine mit (Schaft) Anti-human-Globulin sensibilisierte Polystyrolkugel zugegeben und 1 Stunde bei 37 °C inkubiert. Nach Abtrennung des nicht gebundenen Materials wird die Kugel mit (Ziegen) Anti-human-Globulin-Peroxidase, gelöst in 0,2 M Natriumphosphatpuffer vom pH 6,5 mit 20 % Ziegenserum und 2 g/l Rinderserum-Albumin, während 1 Stunde bei 37 °C inkubiert. Das nicht gebundene Material wird abgetrennt, die Kugel dreimal mit destilliertem Wasser gewaschen und anschliessend zur quantitativen Bestimmung der immunologisch gebundenen Peroxidase in 0,5 ml Substratpuffer (0,1 M Citratpuffer vom pH 5,0 mit 6 mM $H_2O_2$ und 40 mM o-Phenylendiamin) während 30 Minuten bei Raumtemperatur inkubiert. Nach Abstoppen der enzymatischen Reaktion durch Zugabe von 2 ml einer 1 N HCl wird zur Ermittlung des Gehaltes an Humanglobulin die Absorptionsdifferenz bei der Wellenlänge 492 nm photometrisch gemessen. Die Resultate sind in der Tabelle II zusammengefasst.

Tabelle II

| Testbriefchen: | Okkultblut-Test (Farbintensität) | Enzym-immunologischer Test ($\Delta A_{492}$ nm/RT/30 Min.) |
|---|---|---|
| Ohne Stuhlprobe (Reagenzienleerwert) | 0 | 0,000 |
| Stuhlprobe ohne Blut-Zusatz | 0 | 0,070 |
| Stuhlprobe mit 4 ml Human- blut/100 g Stuhl | 2 + | 0,650 |
| Stuhlprobe mit 4 ml Ziegen- blut/100 g Stuhl | 2 + | 0,050 |
| Stuhlprobe mit 4 ml Pferde- blut/100 g Stuhl | 2 + | 0,080 |
| Stuhlprobe mit 4 ml Kanin- chenblut/100 g Stuhl | 2 + | 0,080 |
| Stuhlprobe mit 4 ml Rinder- blut/100 g Stuhl | 2 + | 0,110 |
| Stuhlprobe mit 10 mg Per- oxidase/100 g Stuhl | 4 + | 0,050 |

Aus der Tabelle II geht die Spezifität des enzymimmunologischen Okkultblut-Tests hervor, da nur Proben mit einem Gehalt an Humanblut einen signifikanten Anstieg der Absorptionsdifferenz zeigen, wogegen Proben mit tierischem Blut oder mit Peroxidase (jedoch ohne Humanblut) keinen solchen Anstieg, aber dennoch eine Farbintensität zeigen.

**Patentansprüche**

1. Verfahren zum Nachweis von okkultem Humanblut in Humanstuhlproben nach der Peroxidase-Methode, dadurch gekennzeichnet, dass anschliessend an die Durchführung dieser Methode in derselben Stuhlprobe Humanhämoglobulin, Humanalbumin oder ein Humanglobulin immunologisch nachgewiesen wird.

2. Verfahren nach Anspruch 1, dadurch gekennzeichnet, dass die Peroxidase-Methode mit Benzidin, o-Toluidin oder Guajak als Indikator durchgeführt wird.

3. Verfahren nach Anspruch 1 oder 2, dadurch gekennzeichnet, dass das Humanglobulin Immunglobulin G, Immunglobulin M oder Immunglobulin A ist.

4. Verfahren nach einem der Ansprüche 1 bis 3, dadurch gekennzeichnet, dass der immunologische Nachweis von Humanhämoglobin, Humanalbumin oder Humanglobulin nach dem Sandwich-Verfahren erfolgt.

5. Verfahren nach Anspruch 4, dadurch gekennzeichnet, dass beim Sandwich-Verfahren zwei spezifische Antikörper verwendet werden, welche durch Immunisieren unterschiedlicher Tiere gewonnen werden, von denen einer an eine Festphase gebunden ist und der andere mit einer Markierung versehen ist.

6. Verfahren nach Anspruch 5, dadurch gekennzeichnet, dass die Festphase aus Kuntstoffkügelchen besteht.

7. Verfahren nach Anspruch 5, dadurch gekennzeichnet, dass ein Antikörper mit einem Enzym markiert ist.

8. Verfahren nach Anspruch 7, dadurch gekennzeichnet, dass das Enzym Peroxidase ist.

## Claims

1. A process for the detection of occult human blood in human stool samples according to the peroxidase method, characterized by immunologically detecting human haemoglobin, human albumin or a human globulin in the same stool sample after carrying out this method.

2. A process according to claim 1, characterized in that the peroxidase method is carried out using benzidine, o-toluidine or guaiacum as the indicator.

3. A process according to claim 1 or 2, characterized in that the human globulin is immunoglobulin G, immunoglobulin M or immunoglobulin A.

4. A process according to any one of claims 1 to 3, characterized in that the immunological detection of human haemoglobin, human albumin or human globulin is carried out according to the sandwich method.

5. A process according to claim 4, characterized in that in the sandwich method there are used two specific antibodies, which are obtained by immunizing different animals, of which one is bound to a solid phase and the other is provided with a label.

6. A process according to claim 5, characterized in that the solid phase consists of synthetic beads.

7. A process according to claim 5, characterized in that one antibody is labelled with an enzyme.

8. A process according to claim 7, characterized in that the enzyme is peroxidase.

## Revendications

1. Procédé pour la détection de sang humain indécelable à l'examen direct dans des échantillons de selles humaines par la méthode à la peroxydase, caractérisé en ce que, après la mise en œuvre de cette méthode, et dans le même échantillon de selles, on procède à une recherche immunologique de l'hémoglobine humaine, d'albumine humaine ou d'une globuline humaine.

2. Procédé selon la revendication 1, caractérisé en ce que la méthode à la peroxydase est mise en œuvre avec, en tant qu'indicateur, de la benzidine, de l'ortho-toluidine ou du guajac.

3. Procédé selon la revendication 1 ou 2, caractérisé en ce que la globuline humaine est l'immunoglobuline G, l'immunoglobuline M ou l'immunoglobuline A.

4. Procédé selon l'une des revendications 1 à 3, caractérisé en ce que la recherche immunologique de l'hémoglobine humaine, de l'albumine humaine ou de la globuline humaine est effectuée par le procédé dit « en sandwich ».

5. Procédé selon la revendication 4, caractérisé en ce que, dans le procédé « en sandwich », on utilise deux anticorps spécifiques obtenus par immunisation d'animaux différents, et dont l'un est fixé sur une phase solide et l'autre est marqué.

6. Procédé selon la revendication 5, caractérisé en ce que la phase solide consiste en sphérules de résine synthétique.

7. Procédé selon la revendication 5, caractérisé en ce qu'un anticorps est marqué par une enzyme.

8. Procédé selon la revendication 7, caractérisé en ce que l'enzyme est la peroxydase.